# EUROPÄISCHE PATENTSCHRIFT

(11) **EP 0 906 913 B1**
(45) Veröffentlichungstag und Bekanntmachung des Hinweises auf die Patenterteilung: **23.05.2001**
(21) Anmeldenummer: 97117276.2
(22) Anmeldetag: 06.10.1997
(51) Int. Cl.: C07D 475/00, A61K 31/505

(54) **Pteridinderivate als NO Synthase-Hemmer**
Pteridinderivatives as NO synthase inhibitors
Des dérivés de ptéridin comme inhibiteur de NO synthase

(43) Veröffentlichungstag der Anmeldung: 07.04.1999
(73) Patentinhaber: Werner, Ernst, 6020 Innsbruck (AT)
(72) Erfinder: Werner, Ernst, 6020 Innsbruck (AT); Schircks, Bernhard, 8645 Jona (CH)
(74) Vertreter: Hofinger, Engelbert, Dr.Dr.

(56) Entgegenhaltungen:
- DE-A- 4 418 096
- DE-A- 4 418 097
- DE-A- 19 503 966

## Beschreibung

Die vorliegende Erfindung betrifft Pteridinderivate und deren Verwendung als Pharmaka zur Behandlung von Erkrankungen, die mit einer erhöhten Aktivität von Stickstoffmonoxidsynthasen einhergehen.

Stickstoffmonoxidsynthasen katalysieren die Umwandlung von L-Arginin in L-Citrullin und Stickstoffmonoxid (NO). NO, oder ein Reaktionsprodukt von NO mit z.B. Superoxid oder Thiolen, ist für eine Reihe wichtiger Reaktion im Körper verantwortlich, z.B. zur Abwehr von Pathogenen, zur Regulation des Blutdruckes, zur Übertragung von Nervenreizen. In Erkrankungen kann es zu einer Überproduktion von Stickstoffmonoxid kommen, die mit den Symptomen der Erkrankung direkt in Zusammenhang gebracht werden können. Dazu zählt z.B. der akute Blutdruckabfall während septischer Komplikationen oder während Zytokinbehandlungen, die Entwicklung von Demenzsymptomen in Infektionen mit dem humanen Immunodefizienzvirus (HIV), in der Alzheimer'schen und der Parkinson'schen Erkrankung. In diesen und anderen Erkrankungen mit gesteigerter NO Produktion wird versucht, Pharmaka, die die NO-Synthase Reaktion hemmen können, als neue Therapeutika einzusetzen.

Bisher bekannte Hemmer der NO-Synthasen sind Argininanaloga (z.B. N-Monomethyl-L-Arginin), Derivate von Aminoguanidinen, Thiocitrullin, Isothioharnstoff, Iminoethylornithin, 6-Nitroindazol, 7-Nitroindazol, das 4-Amino-Analogon des Tetrahydrobiopterin (H₄aminobiopterin; Ref. 1), sowie andere Derivate von Pteridinen (DE4418096) und Tetrahydropteridinen (DE 4418097).

Durch die Erfindung wird folgende neue Klasse von Pteridinen zur Verfügung gestellt: 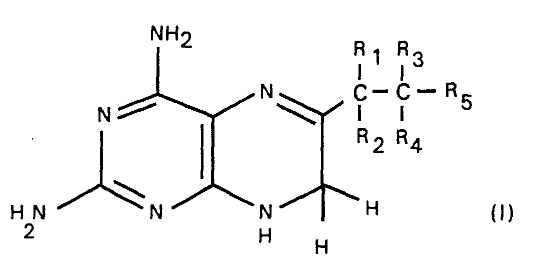 R₁, R₂, R₃, R₄ in Formel (I) stehen unabhängig voneinander für H oder OH, R₅ steht für H, CH₃, CH₂OH oder einen niederen Alkylrest (C1 bis C9), der geradkettig oder verzweigt sein kann, sowie für (CH(OH))ₙ-Y oder (CH(OH))ₙ-(CH₂)ₘ-W, wobei Y Wasserstoff oder ein niederer Alkylrest, W ein Wasserstoff oder eine Hydroxylgruppe ist, und n und m unabhängig voneinander 1-20 sind. Die Erfindung umfaßt weiters alle tautomeren und stereoisomeren Formen von (I), sowie deren Salze.

Die Herstellung dieser noch nicht beschriebenen Verbindungen erfolgt nach bekannten Verfahren. Überraschend wurde gefunden, daß 7,8-Dihydropteridine ausgezeichnete Hemmer von NO-Synthasen sind, die in ihrer Wirkung am Enzym den entsprechenden, bekannten Tetrahydroverbindungen gleichkommen. An intakten, kultivierten Zellen sind aber die Dihydroverbindungen deutlich wirksamer als die entsprechenden Tetrahydroderivate. Zusätzlich zu der im Vergleich zu den Tetrahydroderivaten höheren chemischen Stabilität ist ein weiterer Vorteil der Verwendung der Dihydroderivate darin zu sehen, daß mit weniger Schritten ein einheitliches Produkt hergestellt werden kann. Während bei der Reduktion des entsprechenden "nicht-reduzierten", d.h. voll aromatischen Pteridins zur 5,6,7,8-Tetrahydroverbindung ein Gemisch von Diasteromeren gebildet wird, da an C-6 ein neues chirales Zentrum entsteht, liefert die Herstellung der 7,8-Dihydroverbindung ein einheitliches Produkt. Daher muß nicht der mühsame Weg der Trennung der Isomeren beschritten werden, um nach der chemischen Reduktion eine einheitliche Verbindung zu erhalten.

### Ausführungsbeispiel:

### Herstellung des 2,4-diamino-7,8-dihydro-6(L-erythro-1,2-dihydroxylpropyl) pteridin (H₂aminobiopterin, R₁ = R₃ = H, R₂ = R₄ = OH, R₅ = CH₃):

Die entsprechende "nicht-reduzierte" Verbindung *2,4-Diamino-6-(L -erythro* 1,2,-dihydroxylpropyl)pterin (Aminobiopterin) wird nach bekannten Vorschriften synthetisiert (Ref. 2 - 4). Davon ausgehend wird die 7,8-Dihydroverbindung nach der Methode von Futterman (Ref. 5) mit den Modifikationen von Fukushima & Akino (Ref. 6) hergestellt. Dazu werden 250 mg Aminobiopterin in 50 ml Wasser suspendiert. Nach der Zugabe von 1 g Na₂S₂O₄ wird die Suspension bei 60°C 40 Minuten lang inkubiert. Die Lösung wird dann auf ca 15 ml eingeengt und auf eine Zellulosesäule aufgetragen. Die Säule wird mit Wasser eluiert und die H₂aminobiopterin-hältigen Fraktionen werden bis zum Trocknen lyophilisiert. So erhält man etwa 75 mg H₂aminobiopterin.

Zur Analyse der Verbindung eignet sich z.B. Hochdruckflüssigchromatographie mit den folgenden experimentellen Parametern: Eine 250 mm lange Ionenaustauschersäule mit 4,6 mm innerem Durchmesser (Partisil 10 SCX, Whatman, Maidstone U.K.) wird mit 50 mM Na₂HPO₄, pH = 3, und einer Flußrate von 1,5 ml/min eluiert. Die Verbindungen werden durch UV-Absorption bei 254 nm detektiert. Folgende Retentionszeiten werden beobachtet: 2,4-Diamino-6-(L*-erythro*-1,2 dihydroxylpropyl)pteridin (Aminobiopterin; 5,0 min), 2,4-Diamino-7,8-dihydro-6-(L-*erythro*-1,2 dihydroxylpropyl)pteridin (H₂aminobiopterin; 5,3 min), 2,4-Diamino-5,6,7,8-tetrahydro-6R-(L*-erythro*-1,2 dihydroxypropyl)pteridin (6R H₄ aminobiopterin; 5,6 min), 2,4-Diamino-5,6,7,8-tetrahydro-6S-(L-*erythro*-1,2 dihydroxylpropyl)pteridin (6S H₄aminobiopterin; 6,4 min).

Zum Nachweis der Wirkung der Hemmstoffe eignet sich z.B. rekombinante, neuronale, Tetrahydrobiopterin-freie NO-Synthase, welche nach der Methode von List et al. 1996 (Ref. 7) hergestellt wird. Die NO-Synthaseaktivität wird dabei in Abhängigkeit von der Zugabe des NO-Synthase-Hemmstoffes durch die Bildung von [2,3,4,5-³H]Citrullin aus [2,3,4,5-³H]Arginin nach der Methode von Mayer et al. (Ref. 8) bestimmt. Dazu wird in 0.1 ml folgendes Gemisch für 10 min bei 37°C inkubiert: 0.1 *µ*g NO-Synthase, 0.1mM [2,3,4,5-³H]-L-Arginin (ca. 60000 cpm), 0.5 mM CaCl₂, 10 *µ*g/ml Calmodulin, 0.2 mM NADPH (Nicotinamidadenindinukleotid, reduzierte Form), 10 *µ*M Tetrahydrobiopterin, 0-1000 µM NO-Synthase-Inhibitor (z.B. H₂aminobiopterin), 5 *µ*M FAD (Flavinadenindinucleutid), 5 µM FMN (Flavinmononucleotid), und 0.2 mM CHAPS (3-[(3-cholamidopropyl)dimethylamonio]-1-propansulfonat). Die Reaktion wird durch Zugabe von 0.9 ml 20 mM Natriumacetatpuffer, pH = 5,5, der 1 mM L-Citrullin enthält, gestoppt. Das Gemisch wird anschließend auf Ionenaustauschersäulen, die mit dem Material DOWEX AG50W-X8 gefüllt sind, aufgetragen, und das Eluat sowie die Waschflüssigkeit (1 ml Wasser) werden aufgefangen. 7 ml Scintillationscocktail werden zugegeben und die Radioaktivität, die ein Maß für die NO-Synthaseaktivität ist, wird mit einem Szintillationszähler bestimmt.

**Tabelle I**

| Vergleich der Wirksamkeit von H₂ aminobiopterin (R₁ = R₃ = H, R₂ = R₄ = OH, R₅ = CH₃ ), H₄ aminobiopterin und Beispiel 72 aus DE4418097 an rekombinanter, Tetrahydrobiopterin-freier NO-Synthase | | | |
|---|---|---|---|
| Inhibitor | Konzentration (µM) | NO-Synthase -Aktivität (nmol.mg ⁻¹ min ⁻¹ ) | Hemmung (%) |
| Keiner | | 295 | 0 |
| H₂aminobiopterin | 0.01 | 294 | 0.3 |
| H₂aminobiopterin | 0.1 | 283 | 4 |
| H₂aminobiopterin | 1.0 | 242 | 18 |
| H₂aminobiopterin | 10. | 110 | 64 |
| H₂aminobiopterin | 100. | 48 | 83 |
| H₂aminobiopterin | 1000. | 25 | 92 |
| Keiner | | 298 | 0 |
| H₄aminobiopterin | 0.01 | 279 | 6 |
| H₄aminobiopterin | 0.1 | 262 | 12 |
| H₄aminobiopterin | 1.0 | 181 | 39 |
| H₄aminobiopterin | 10. | 72 | 76 |
| H₄aminobiopterin | 100. | 35 | 88 |
| H₄aminobiopterin | 1000. | 24 | 92 |
| Keiner | | 334 | 0 |
| DE 4418097-72 | 0.01 | 325 | 3 |
| DE 4418097-72 | 0.1 | 318 | 5 |
| DE 4418097-72 | 1.0 | 306 | 8 |
| DE 4418097-72 | 10. | 316 | 5 |
| DE 4418097-72 | 100. | 274 | 18 |
| DE 4418097-72 | 1000. | 136 | 60 |

Tabelle 1 zeigt, daß H₂aminobiopterin das Enzym in der gleichen Größenordnung hemmen kann wie der beste bisher bekannte Hemmer der NO-Synthase auf Pteridinbasis, 6R-H₄aminobiopterin. Überdies ist die Verbindung wesentlich wirksamer als bisher patentierte Hemmer der NO-Synthase auf Pteridinbasis, wie der Vergleich mit Beispiel 72 aus DE 4418097 zeigt.

Zum Nachweis der Wirksamkeit einer Verbindung der vorliegenden Erfindung an kultivierten Zellen wurden Mausfibroblasten nach Werner-Felmayer et al., (Ref. 9) gewonnen und in RPMI 1640 Medium (Schoeller Pharma, Wien, Österreich) mit 20% Hitze-inaktiviertem fötalem Kälberserum (Schoeller Pharma) in einer Dichte von 10⁵ Zellen pro ml ausgesät und mit 50 U/ml Mäuse-Interferongamma, 100 U/ml Tumornekrose Faktor-Alpha und 1 *µ*g/ml Lipopolysaccharid (E. coli B55:05) in Gegenwart verschiedener Konzentrationen von Hemmstoffen kultiviert. Nach 96 h im Brutschrank (100% Luftfeuchtigkeit, 37°C, 5% CO₂) werden die Überstände gesammelt und mit der Griess-Reaktion auf ihren Gehalt an Nitrit, einem Abbauprodukt von NO, analysiert. Die folgende Tabelle 2 zeigt einen Vergleich von H₂aminobiopterin (R₁ = R₃ = H, R₂ = R₄ = OH, R₅ = CH₃), H₄aminobiopterin und Beispiel 72 aus DE 4418097:

**Tabelle 2**

| Vergleich von H₂ aminobiopterin (R₁ = R₃ = H, R₂ = R₄ = OH, R₅ = CH₃ ), H ₄ aminobiopterin und DE 4418097-72 an kultivierten, cytokin-stimulierten Mausfibroblasten: | | | |
|---|---|---|---|
| Inhibitor | Konzentration (µM) | Nitrit im Überstand (µM) | Hemmung (%) |
| Keiner | | 9.5 | 0 |
| H₂aminobiopterin | 2.5 | 6.2 | 35 |
| H₂aminobiopterin | 10 | 2.1 | 78 |
| H₂aminobiopterin | 25 | 0.1 | 99 |
| H₂aminobiopterin | 100 | 0.0 | 100 |
| H₂aminobiopterin | 250 | 0.0 | 100 |
| Keiner | | 10.0 | 0 |
| H₄aminobiopterin | 2.5 | 9.3 | 7 |
| H₄aminobiopterin | 10 | 6.4 | 36 |
| H₄aminobiopterin | 25 | 3.8 | 62 |
| H₄aminobiopterin | 100 | 0.4 | 96 |
| H₄aminobiopterin | 250 | 0.02 | 99 |
| Keiner | | 9.8 | 0 |
| DE 4418097-72 | 2.5 | 9.8 | 0 |
| DE 4418097-72 | 10 | 9.1 | 7 |
| DE 4418097-72 | 25 | 8.6 | 12 |
| DE 4418097-72 | 100 | 5.8 | 41 |
| DE 4418097-72 | 250 | 1.8 | 82 |

Tabelle 2 zeigt klar, daß H₂aminobiopterin an kultivierten Zellen deutlich wirksamer ist als bisher bekannte Pteridinderivate.

Die Dosierung der Hemmstoffe mit der Formel (I) kann in einem weiten Bereich schwanken. Therapeutisch eingesetzt werden Konzentrationen etwa 20 bis 100 *µ*M, sowie etwa 1 bis 100 mg/kg, entweder oral oder als Bolusinjektion (etwa 2-30 mg/kg), als Depot oder als kontinuierliche Infusion (etwa 0.2 - 3 mg/kg/h). Als Vehikel für orale Gabe eignen sich z.B. Tabletten die 5-20% Wirkstoff, gemeinsam mit einer äquimoler Menge L-Ascorbinsäure, einer halben äquimolaren Menge N-Acetylcystein und dem Rest pharmazeutische Hilfsstoffe enthalten. Präparate können auch Gemische von verschiedenen Verbindungen der Erfindung enthalten.

Verschiedene Krankheitsbilder können verschieden auf Veränderungen im NO Spiegel bzw. der NO Synthase Aktivität reagieren. Asthma, z.B., kann durch eine relative geringfügige (5-10%ige) Verringerung der NO Spiegel bzw. der NO Synthaseaktivität deutlich verbessert werden, während z.B. die Abschwächung neurologischer Degenerationen mehr als 20% Hemmung der NO Synthaseaktivität bzw. Senkung der NO Spiegel erfordern kann.

### Referenzen:

1) E.R. Werner, E. Pitters, K. Schmidt, H. Wachter, G. Werner-Felmayer & B. Mayer. Identification of the 4-amino analogue of tetrahydrobiopterin as a dihydropteridine reductase inhibitor and potent pteridine antagonist of rat neuronal nitric oxide synthase. Biochem. J. 320:193-196 (1996)
2) B. Schircks, J.H. Bieri & M. Viscontini, Eine neue, regiospezifische Synthese von L-Biopterin. Helv. Chim. Acta. 60 (Fasc. 1) 211-214 (1977)
3) B. Schircks, Neue Regiospezifische Synthese von L-Biopterin und dessen Derviaten. Ph.D. Thesis, University of Zürich, Switzerland, 1978.
4) B. Schircks, J.H. Bieri & M. Viscontini, Helv. Chim. Acta. 68, 1639 - 1643 (1985)
5) S. Futterman, Enzymatic reduction of folic acid and dihydrofolic acid to tetrahydrofolic acid. J. Biol. Chem 228, 1031-1038 (1957)
6) T. Fukishima & M. Akino, Nuclear magnetic resonance studies of some biologically active dihydropterins. Arch. Biochem. Biophys. 128, 1-5 (1968)
7) B.M. List, P. Klatt, E.R. Werner, K. Schmidt & Mayer, B. Overexpression of neuronal nitric oxide synthase in insect cells reveals requirement of heme for tetrahydrobiopterin binding. Biochem. J. 315, 57-63 (1996)
8) B. Mayer, P. Klatt, E. R. Werner, and Schmidt, K. Molecular mechanisms of inhibition of porcine brain nitric oxide synthase by the antinociceptive drug 7-nitro-indazole. Neuropharmacology. 33:1253-1259, (1994)
9) G. Werner-Felmayer G, E.R. Werner, D. Fuchs, A. Hausen, G. Reibnegger, H. Wachter. Tetrahydrobiopterin-dependent formation of nitrite and nitrate in murine fibroblasts. J. Exp. Med. 172:1599-1607 (1990)

## Patentansprüche

1. Pteridinderivat der Formel 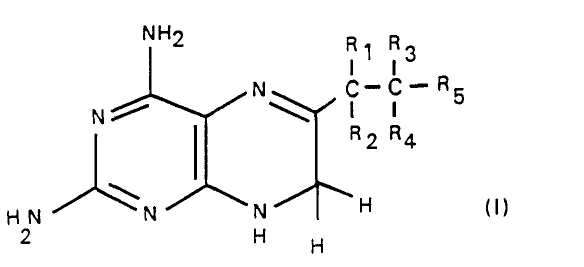 wobei R₁, R₂, R₃, R₄ unabhängig voneinander für H oder OH stehen, R₅ für H, CH₃, CH₂OH oder einen niederen Alkylrest (C1 bis C9) steht, der geradkettig oder verzweigt sein kann, sowie für (CH(OH))ₙ-Y oder (CH(OH))ₙ-(CH₂)ₘ-W, wobei Y Wasserstoff oder ein niederer Alkylrest, W ein Wasserstoff oder eine Hydroxylgruppe ist, und n und m unabhängig voneinander 1-20 sind, sowie tautomere und stereoisomere Formen von (I) und deren Salze.

2. Pteridinderivat nach Anspruch 1, dadurch gekennzeichnet, daß R₅ für Methyl, R₃ für Wasserstoff, R₄ für Hydroxyl, R₂ für Hydroxyl und R₁ für Wasserstoff oder Hydroxyl stehen.

3. Arzneimittel dadurch gekennzeichnet, daß es ein Pteridinderivat nach Anspruch 1 oder 2 als Wirkstoff enthält.

4. Verwendung eines Pteridinderivates nach Anspruch 1 oder 2, zur Herstellung eines Heilmittels gegen eine Krankheit, die mit gesteigerter NO-Erzeugung verbunden ist.

## Claims

1. A pteridine derivative of the formula: 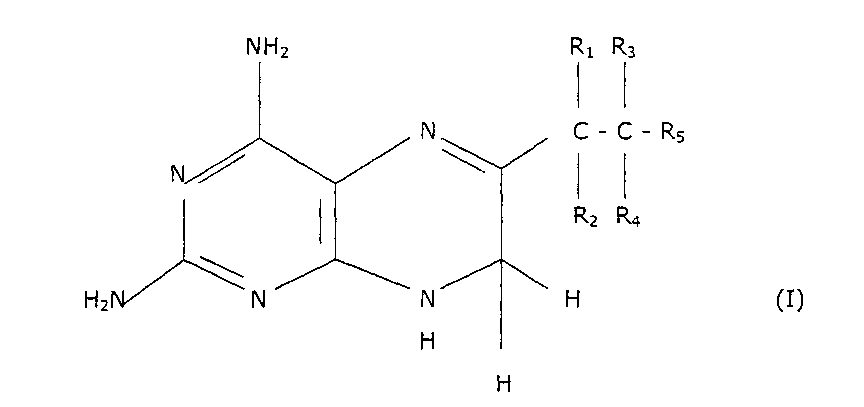 wherein R₁, R₂, R₃ and R₄ independently of each other stand for H or OH, R₅ stands for H, CH₃, CH₂OH or a low alkyl residue (C1 to C9) which can be straight-chain or branched, and for (CH(OH))ₙ-Y or (CH(OH))ₙ-(CH₂)ₘ-W, wherein Y is hydrogen or a. low alkyl residue, W is hydrogen or a hydroxyl group, and n and m independently of each other are 1 - 20, as well as tautomeric and stereoisomeric forms of (I) and salts thereof.

2. A pteridine derivative according to claim 1 characterised in that R₅ stands for methyl, R₃ stands for hydrogen, R₄ stands for hydroxyl, R₂ stands for hydroxyl and R₁ stands for hydrogen or hydroxyl.

3. A medicament characterised in that it contains a pteridine derivative according to claim 1 or claim 2 as an active substance,

4. Use of a pteridine derivative according to claim 1 or claim 2 for the production of a remedy for a disease which is linked to increased NO-production.

## Revendications

1. Dérivé de ptéridine de la formule : 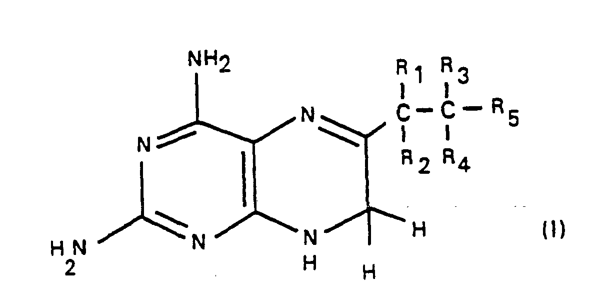 dans laquelle R₁, R₂, R₃, R₄ représentent indépendamment les uns des autres H ou OH, R₅ représente H, CH₃, CH₂OH ou un radical alkyle inférieur (C1 à C9) qui peut être à chaîne linéaire ou ramifiée, ainsi que (CH(OH))ₙ-Y ou (CH(OH))ₙ-(CH₂)ₘ-W, où Y est l'hydrogène ou un radical alkyle inférieur, W est un hydrogène ou un groupe hydroxyle, et n et m sont indépendamment l'un de l'autre 1-20, ainsi qùe les formes tautomères et stéréo-isomères de (I) et leurs sels.

2. Dérivé de ptéridine selon la revendication 1, caractérisé en ce que R₅ représente méthyle, R₃ hydrogène, R₄ hydroxyle, R₂ hydroxyle et R₁ hydrogène ou hydroxyle.

3. Médicament, caractérisé en ce qu'il contient un dérivé de ptéridine selon la revendication 1 ou 2 en tant que principe actif.

4. Utilisation d'un dérivé de ptéridine selon la revendication 1 ou 2 pour la fabrication d'un remède contre une maladie qui est liée à une génération accrue de NO.
